(19) Europäisches Patentamt
European Patent Office
Office européen des brevets

(11) EP 0 826 661 A2

(12) **EUROPÄISCHE PATENTANMELDUNG**

(43) Veröffentlichungstag:
**04.03.1998 Patentblatt 1998/10**

(51) Int. Cl.$^6$: **C07C 229/22**, A61K 7/06

(21) Anmeldenummer: **97110954.1**

(22) Anmeldetag: **02.07.1997**

(84) Benannte Vertragsstaaten:
**AT BE CH DE DK ES FI FR GB GR IE IT LI LU MC NL PT SE**
Benannte Erstreckungsstaaten:
**AL LT LV RO SI**

(30) Priorität: **06.08.1996 DE 19631685**

(71) Anmelder:
**Wella Aktiengesellschaft
64274 Darmstadt (DE)**

(72) Erfinder:
- **kripp, Thomas, Dr.
64407 Fränkisch-Crumbach (DE)**
- **Czigler, Thomas
64347 Griesheim (DE)**
- **Lang, Günther, Dr.
64354 Reinheim (DE)**
- **Bimczok, Rudolf, Dr.
64342 Seeheim (DE)**

(54) **Hydrolytisch spaltbare Wirkstoffderivate, diese enthaltende Haarbehandlungsmittel**

(57) Gegenstand der vorliegenden Anmeldung sind neue Wirkstoffderivate der Formel (I)

$$[R^2-\overset{\displaystyle R^1}{\underset{\displaystyle R^3}{\overset{|}{\underset{|}{N^+}}}}-Y-COOR]\ A^- \qquad (I),$$

mit $R^1$, $R^2$ und $R^3$ unabhängig voneinander gleich einem unsubstituierten oder substituierten Alkylrest, Y gleich einem substituierten oder unsubstituierten Alkylrest, R gleich einem Wirkstoff mit mindestens einer OH-Gruppe im Molekül und $A^-$ gleich einem Anion, ein Verfahren zur Herstellung der Verbindungen der Formel (I) sowie diese Verbindungen enthaltende Haarbehandlungsmittel und ein Verfahren zum Behandeln von Haaren.

EP 0 826 661 A2

Printed by Xerox (UK) Business Services
2.15.12/3.4

## Beschreibung

Die vorliegende Erfindung betrifft hydrolytisch spaltbare Wirkstoffderivate, diese enthaltende Haarbehandlungsmittel sowie ein Verfahren zum Behandeln von Haaren.

Zum Zwecke der Verschönerung und Pflege der Haare oder zum Schutz der Haare werden eine Vielzahl verschiedenartiger Wirkstoffe eingesetzt. Einige dieser Wirkstoffe wirken nur während der kurzen Applikationsdauer (wie zum Beispiel Dauerwellmittel) und werden sodann aus dem Haar wieder ausgespült, andere Wirkstoffe verbleiben im Haar und behalten ihre Wirkung für eine gewisse Zeit. Als Beispiele für die letztgenannten Wirkstoffe können Haarfestiger und Haarsprays, welche ihre Wirkung bis zum nächsten Kontakt mit Feuchtigkeit beibehalten, oder Haarspülungen und Haarkuren, welche bis zur nächsten Haarwäsche im Haar verbleiben, oder temporäre beziehungsweise semipermanente Haarfärbemittel ("Haartönungen"), welche je nach ihrer Haltbarkeit über einen mehr oder weniger langen Zeitraum im Haar verbleiben, wobei jedoch die Intensität der Haarfärbungen mit jeder Haarwäsche abnimmt, genannt werden.

In steigendem Maße besteht das Bedürfnis, die Wirkungsdauer von in Haarbehandlungsmitteln enthaltenen Wirkstoffen zu verlängern, um so einen Langzeitschutz beziehungsweise eine Langzeitpflege der Haare zu ermöglichen. Besonderer Wert wird hierbei auf eine hohe Auswaschresistenz der Wirkstoffe gelegt, damit die Wirkstoffe ihre Wirkung auch nach mehrmaligen Haarwäschen beibehalten.

Weiterhin besteht ein dringendes Bedüfnis, auch wenig oder gar nicht wasserlösliche Wirkstoffe sowie Wirkstoffe mit einem geringen Penetrationsvermögen der Verwendung in kosmetischen Mitteln zugänglich zu machen.

Aufgabe der vorliegenden Erfindung ist es daher, neue Wirkstoffderivate zur Verfügung zu stellen, mit denen die vorgenannten Anforderungen weitestgehend erfüllt werden.

Gegenstand der vorliegenden Erfindung sind daher hydrolytisch spaltbare Wirkstoffderivate der Formel (I)

$$\left[R^2-\overset{\overset{\displaystyle R^1}{|}}{\underset{\underset{\displaystyle R^3}{|}}{N^+}}-Y-COOR\right]\ A^- \quad (I),$$

mit $R^1$, $R^2$ und $R^3$ unabhängig voneinander gleich einem unsubstituierten oder substituierten Alkylrest, Y gleich einem substituierten oder unsubstituierten Alkylrest, R gleich einem Wirkstoffradikal, das vor der Esterbildung mindestens eine OH-Gruppe im Molekül aufweist und $A^-$ gleich einem Anion.

Als Reste $R^1$, $R^2$ und/oder $R^3$ können vorzugsweise $C_1$- bis $C_6$- Alkylreste und insbesondere $C_1$- bis $C_4$- Alkylreste, beispielsweise Methyl-, Ethyl-, Propyl-, Isopropyl- oder Butylreste, wobei der Methylrest besonders bevorzugt ist, genannt werden. Die vorgenannten Alkylreste können auch mit einer oder mehreren Hydroxy- oder Aminogruppen substituiert sein.

Der Rest Y stellt vorzugsweise einen unsubstituierten $C_1$- bis $C_6$- Alkylrest, insbesondere eine $-CH_2-$, $-CH_2-CH_2-$, $-CH_2-CH_2-CH_2-$, $-CH_2-CH_2-CH_2-CH_2-$Gruppe, oder einen mit einer oder mehreren Hydroxy-, Amino-, Acyl-(insbesondere $C_1$- bis $C_2$- Acylgruppen) oder quaternäre Ammoniumgruppen substituierten $C_2$- bis $C_{10}$- Alkylrest, insbesondere eine substituierte $-CH_2-CH_2-$,$-CH_2-CH_2-CH_2-$, $-CH_2-CH_2-CH_2-CH_2-$Gruppe, dar.

Als geeignetes Wirkstoffradikal R kann jeder vor der Esterbildung mindestens eine OH-Gruppe enthaltende Wirkstoffrest verwendet werden, wobei neben üblichen Pflegestoffen auch wasserunlösliche beziehungsweise in Wasser nur wenig lösliche Verbindungen, wie zum Beispiel organophile, niedermolekulare Wirkstoffe mit mindestens einer freien OH-Gruppe im Molekül, und Verbindungen mit einem geringen Penetrationsvermögen eingesetzt werden können.

Als Beispiele für geeignete Wirkstoffe können genannt werden: Pflegestoffe (zum Beispiel Fettalkohole, Panthenol, Cholesterin oder Farnesol), Vitamine (beispielsweise Vitamin A (Retinol), Vitamin $A_2$ (Didehydroretinol), Vitamin $D_2$ (Calciferol), Vitamin E (Tocopherol), Vitamin $K_1$ oder $K_2$), Duftstoffe (zum Beispiel Ethylvanillin, Geraniol, Nerol, Linalool, $\alpha$-Terpineol oder Menthol), Konservierungsstoffe beziehungsweise fungizide oder bakterizide Wirkstoffe (zum Beispiel Thymol oder p-Hydroxybenzoesäureester), Lipide (beispielsweise Diglyceride) oder Farbstoffe.

Als Wirkstoffderivate der Formel (I) sind insbesondere die Wirkstoffester des L-, D- oder L,D-Carnitins ($R^1$, $R^2$, $R^3$ = $-CH_3$ und Y = $-CH_2-CH(OH)-CH_2-$), des L-, D- oder L,D-O-Acetyl-Carnitins ($R^1$, $R^2$, $R^3$ = $CH_3$ und Y = $-CH_2-CH(OOC-$

$CH_3$)-$CH_2$-) oder des Betains ($R^1$, $R^2$, $R^3$ = $CH_3$ und Y = -$CH_2$-) zu nennen, wobei als Anion $A^-$ vorzugsweise ein Sulfation, Phosphation, Hydrogenphosphation, Carbonation, Hydrogencarbonation, Jodidion, Chloridion, Bromidion, Oxalation, Formiation, Acetation, Zitration, Tartration, Malation oder Pyruvation verwendet wird.

Die erfindungsgemäßen Wirkstoffderivate der Formel (I) sind auf einfache Weise durch Veresterung der Verbindungen der Formel (II), wobei $R^1$, $R^2$, $R^3$, Y und $A^-$ die gleiche Bedeutung wie in Formel (I) besitzen,

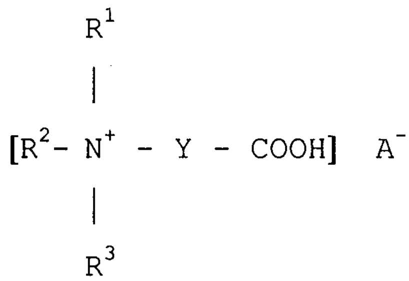

(II),

mit einem Wirkstoff, welcher mindestens eine OH-Gruppe im Molekül aufweist, synthetisierbar.

Hierzu wird die Verbindung der Formel (II) zunächst durch Überführung in das Säurechlorid der Formel (III) aktiviert und sodann mit dem Wirkstoff verestert.

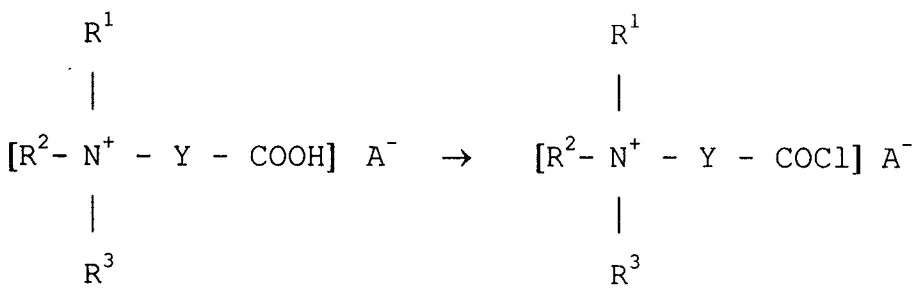

(II) (III)

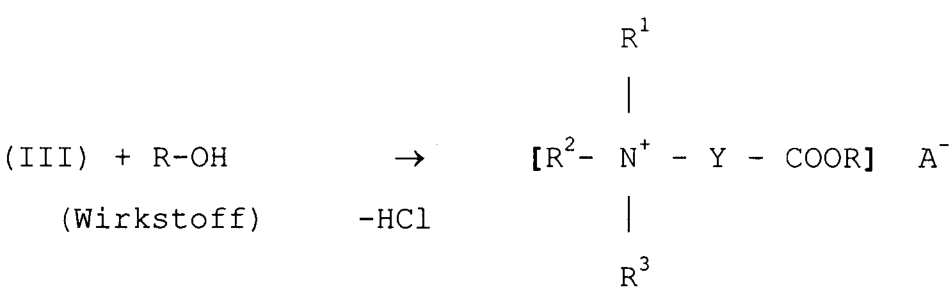


(I)

Die erfindungsgemäßen Wirkstoffderivate der Formel (I) ermöglichen es, wenig oder gar nicht wasserlösliche Wirkstoffe sowie Wirkstoffe mit einem geringen Penetrationsvermögen ins Haar einzuschleusen und so eine wesentlich verbesserte Wirkungsdauer und Auswaschstabilität der Haarbehandlungsmittel zu erzielen. Weiterhin ermöglichen die Wirkstoffderivate der Formel I bei sehr leicht auswaschbaren Wirkstoffen, wie zum Beispiel Rutin, eine deutliche Verbesserung der Wirkungsdauer und eine Erhöhung der Auswaschstabilität.

Ein weiterer Gegenstand der vorliegenden Erfindung ist daher ein Mittel zur Haarbehandlung, welches dadurch gekennzeichnet ist, daß es mindestens ein Wirkstoffderivat der Formel (I) enthält.

Die Wirkstoffderivate der Formel (I) sind in dem erfindungsgemäßen Haarbehandlungsmittel vorzugsweise in einer Gesamtmenge von 0,01 bis 20 Gewichtsprozent, insbesondere 0,1 bis 10 Gewichtsprozent, enthalten.

Das erfindungsgemäße Haarbehandlungsmittel weist einen neutralen bis sauren pH-Wert auf (pH = ≤ 7), wobei ein pH-Wert von 2 bis 7 und insbesondere von 5 bis 6,5 bevorzugt ist.

Für die Einstellung des erfindungsgemäßen neutralen bis sauren pH-Wertes sind sowohl organische als auch anorganische Säuren, beispielsweise $\alpha$-Hydroxycarbonsäuren, wie zum Beispiel Glykolsäure, Milchsäure, Weinsäure, Zitronensäure oder Äpfelsäure, Ascorbinsäure, Gluconsäurelacton, Essigsäure, Salzsäure oder Phosphorsäure, sowie Mischungen der genannten Säuren, geeignet.

Die Einsatzmenge der vorgenannten Säuren liegt vorzugsweise bei 0,1 bis 10 Gewichtsprozent, wobei eine Einsatzmenge von 0,5 bis 3 Gewichtsprozent besonders bevorzugt ist.

Gegebenenfalls kann das erfindungsgemäße Haarbehandlungsmittel zusätzlich zu den Wirkstoffderivaten der Formel (I) auch weitere für Haarbehandlungsmittel übliche Inhaltsstoffe enthalten; beispielsweise Parfümöle; Komplexbildner; Wachse; Konservierungsstoffe, kosmetische Harze, wie zum Beispiel Polyvinylpyrrolidon oder Polyvinylacetat; Verdicker; Alginate; Guar Gum; haarpflegende Substanzen, wie zum Beispiel kationische Polymere oder Lanolinderivate; oder Netzmittel und Emulgatoren aus den Klassen der anionischen, nichtionischen, amphoteren oder kationischen oberflächenaktiven Substanzen.

Die erwähnten Bestandteile werden in den für solche Zwecke üblichen Mengen verwendet, zum Beispiel die Netzmittel und Emulgatoren in einer Konzentration von 0,1 bis 30 Gewichtsprozent, und die Pflegestoffe in einer Menge von 0,1 bis 5 Gewichtsprozent.

Das erfindungsgemäße Haarbehandlungsmittel kann neben Wasser weitere Lösungsmittel, wie zum Beispiel aliphatische Alkohole, insbesondere Ethanol oder Isopropanol, oder Glykolether, insbesondere 1,2-Propandiol, enthalten, wobei der Wassergehalt in der Regel etwa 25 bis 95 Gewichtsprozent, vorzugsweise 30 bis 85 Gewichtsprozent, beträgt, während der Gehalt an der übrigen Lösungsmitteln bei etwa 5 bis 30 Gewichtsprozent liegt.

Das erfindungsgemäße Haarbehandlungsmittel kann in Form einer wäßrigen oder wäßrig-alkoholischen Lösung, einer Creme, eines Geles, einer Emulsion, eines Aerosolsprays oder Aerosolschaumes vorliegen, wobei das Haarbehandlungsmittel sowohl in Form eines Einkomponentenpräparates als auch in Form eines Mehrkomponentenpräparates konfektioniert sein kann.

Besonders bevorzugt ist hierbei die Konfektionierung in Form eines Zweikomponentenpräparates, bei dem das Wirkstoffderivat der Formel (I) getrennt von den übrigen Bestandteilen abgepackt wird und die Herstellung des gebrauchsfertigen Haarbehandlungsmittels erst unmittelbar vor der Anwendung durch Vermischen der beiden Komponenten erfolgt.

Die Anwendung des vorstehend beschriebenen erfindungsgemäßen Haarbehandlungsmittels erfolgt indem man eine für die Haarbehandlung ausreichende Menge des Haarbehandlungsmittels auf das Haar aufträgt, das Haarbehandlungsmittel bei 15 bis 50 Grad Celsius etwa 1 bis 60 Minuten, vorzugsweise 2 bis 30 Minuten, einwirken läßt, sodann das Haar mit einer alkalischen Lösung (pH > 7, vorzugsweise pH = 8 bis 9) behandelt und nach einer kurzen Einwirkungszeit der alkalischen Lösung (etwa 1 bis 15 Minuten, vorzugsweise 2 bis 10 Minuten) das Haar gründlich mit Wasser ausspült, gegebenenfalls mit einem Shampoo wäscht und abschließend trocknet.

Als alkalische Lösung sind insbesondere 0,5- bis 10prozentige, vorzugsweise 1- bis 6prozentige wäßrige Lösungen der folgenden Verbindungen geeignet: Ammoniak, Guanidin, Ammonium- oder Alkalicarbonat (zum Beispiel Natriumcarbonat), Ammonium- oder Alkalihydrogencarbonat (zum Beispiel Natriumhydrogencarbonat).

Bei Verwendung von Wirkstoffderivaten der Formel (I), bei denen die Wirkstoffgruppe eine relativ hohe Wasserlöslichkeit besitzt, kann auch auf die Behandlung der Haare mit der alkalischen Lösung, durch die der Wirkstoff von dem Wirkstoffderivat der Formel (I) hydrolytisch abgespalten wird, verzichtet werden, um so die Fixierung des Wirkstoffes durch ionische Bindung zu unterstützen.

Das erfindungsgemäße Haarbehandlungsmittel ermöglicht eine hervorragende, gleichmäßige, intensive und äußerst dauerhafte Anlagerung von Wirkstoffen an das Haar, wobei der pflegende, festigende, schützende oder färbende Effekt der Wirkstoffe bis zu zehn oder mehr Haarwäschen überdauert.

Die nachfolgenden Beispiele sollen den Gegenstand der Erfindung erläutern, ohne diesen hierauf zu beschränken.

**Beispiele**

**Beispiel 1:** Haarbehandlungsmittel (gelförmig)

| | |
|---|---|
| 2,5 g | Wirkstoffderivat der Formel (I) mit $R^1$, $R^2$, $R^3$ = $-CH_3$; Y = $-CH_2-CH(OOC-CH_3)-CH_2-$; $A^-$ = $Cl^-$ und R = Wirkstoff gemäß Tabelle 1 |
| 1,0 g | Hydroxypropylmethylcellulose |

| | |
|---|---|
| 0,5 g | Laurylpyridiniumchlorid |
| 0,3 g | Parfüm |
| 95,7 g | Wasser |
| 100,0 g | |

Der pH-Wert des Haarbehandlungsmittels wird mit 10prozentiger Zitronensäure auf 6,0 eingestellt.

Tabelle 1

| Beispiel | in Formel (I) für R verwendeter Wirkstoff |
|---|---|
| 1a | Laurylalkohol (Dodecanol) |
| 1b | Myristylalkohol (Tetradecanol) |
| 1c | Cetylalkohol (Hexadecanol) |
| 1d | Vitamin A (Retinol) |
| 1e | Vitamin E (Tocopherol) |
| 1f | Panthenol |
| 1g | Vitamin P (Rutin) |

**A) Beispiele 1a) bis 1e)**

Das Haarbehandlungsmittel wird auf das Haar aufgetragen und 30 Minuten bei 45 °Celsius einwirken gelassen. Sodann wird das Haar 5 Minuten lang mit einer 2prozentigen wäßrigen Ammoniaklösung behandelt und anschließend mit lauwarmem Wasser ausgespült, mit einem Shampoo gewaschen, nochmals mit Wasser ausgespült und getrocknet.

Die pflegende Wirkung bleibt mehrere Haarwäschen ohne einen merkbaren Intensitätsverlust bestehen.

**B) Beispiele 1f) und 1g)**

Das Haarbehandlungsmittel wird auf das Haar aufgetragen und 30 Minuten bei 45 °Celsius einwirken gelassen. Anschließend wird das Haar mit lauwarmem Wasser ausgespült, mit einem Shampoo gewaschen, nochmals mit Wasser ausgespült und getrocknet.

Die pflegende Wirkung des Haarbehandlungsmittels ist auch durch mehrmalige Haarwäsche nur geringfügig vermindert worden.

**Beispiel 2:** Haarbehandlungsmittel (2-Komponenten-Verpackung)

**Komponente 1**

| | |
|---|---|
| 2,1 g | Natrium-cocoamphoacetat (40prozentige wäßrige Lösung |
| 94,9 g | Wasser |
| 97,0 g | |

**Komponente 2**

3,0 g Wirkstoffderivat der Formel (I) mit $R^1$, $R^2$, $R^3$ = $-CH_3$; Y = $-CH_2-CH(OOC-CH_3)-CH_2-$;
$A^-$ = $Cl^-$ und R = Cholesterin

Die beiden Komponenten werden unmittelbar vor der Anwendung miteinander vermischt. Das Haar wird mit dem so erhaltenen gebrauchsfertigen Haarbehandlungsmittel sodann in der in Beispiel 1A) beschriebenen Weise behandelt.

**Beispiel 3:** Synthese von Carnityl-Wirkstoffderivaten

**1. Stufe: Aktivierung des Carnitins**

19,7 g (0,1 mol) Carnitinhydrochlorid werden in 500 ml wasserfreiem Dichlormethan suspendiert und unter Rühren tropfenweise mit 11,9 g (0,15 mol) Thionylchlorid versetzt. Das allmählich entstehende Säurechlorid geht in Lösung. Das Lösungsmittel sowie unumgesetztes Thionylchlorid werden abdestilliert. Es wird ein Gemisch aus Carnitylchlorid und oligo-O-Carnitinyl-Carnitinylchloriden erhalten, das ohne weitere Aufarbeitung in Stufe 2 eingesetzt werden kann.

**2. Stufe: Veresterung des Carnitins zum Carnitinyl-Wirkstoffderivat (allgemeine Vorschrift)**

0,1 mol des zu derivatisierenden Wirkstoffes wird in 500 ml wasserfreiem Dichlormethan gelöst und unter Rühren mit einem pro freier OH-Gruppe des Wirkstoffes 1,5fachen Überschuß an dem Carnitinylchlorid-Gemisch aus Stufe 1 versetzt. Nach 6stündigem Rühren des Reaktionsgemisches bei Raumtemperatur werden 20 ml wasserfreies Methanol zugesetzt, um überschüssiges Carnitinylchlorid-Gemisch zu binden. Die Reaktionsmischung wird sodann weitere 5 Minuten gerührt. Anschließend werden die flüchtigen Bestandteile abdestilliert. Der erhaltene Rückstand kann ohne weitere Reinigung direkt in Haarbehandlungsmitteln eingesetzt werden.

**Beispiel 4:** Synthese von O-Acetylcarnitinyl-Wirkstoffderivaten

**Stufe 1: Darstellung von O-Acylcarnitin (allgemeine Vorschrift)**

Die Darstellung des O-Acylcarnitins erfolgt nach üblichen Acylierungsmethoden mit aktivierten Säuren.

19,7 g (0,1 mol) Carnitinhydrochlorid werden in 100 ml wasserfreier Essigsäure bei 40 °Celsius gelöst beziehungsweise suspendiert. Die erhaltene Reaktionsmischung wird langsam mit 0,1 mol des entsprechenden Säurechlorides ($C_2$- bis $C_4$- Carbonsäurechlorid) versetzt und sodann 3 bis 4 Stunden bei 40 °Celsius gerührt. Anschließend wird das Lösungsmittel sowie unumgesetztes Säurechlorid abdestilliert, der Rückstand in 100 ml wasserfreiem, heißen Isopropanol aufgenommen und heiß filtriert.

Nach Eindampfen des Filtrates wird das O-Acylcarnitin erhalten.

Ausbeute(O-Acetylcarnitinhydrochlorid): 98 % der Theorie.

**2. Stufe: Aktivierung des Carnitins**

23,9 g (0,1 mol) O-Acetylcarnitinhydrochlorid werden mit 500 ml wasserfreiem Dichlormethan suspendiert und unter Rühren tropfenweise mit 11,9 g (0,15 mol) Thionylchlorid versetzt. Das allmählich entstehende Säurechlorid geht in Lösung. Das Lösungsmittel sowie unumgesetztes Thionylchlorid werden abdestilliert. Der erhaltene Rückstand kann ohne weitere Reinigung in Stufe 3 eingesetzt werden.

**3. Stufe: Veresterung des O-Acylcarnitins zum O-Acylcarnitinyl-Wirkstoffderivat (allgemeine Vorschrift)**

0,1 mol des zu derivatisierenden Wirkstoffes wird in 500 ml wasserfreiem Dichlormethan gelöst und unter Rühren mit einem pro freier OH-Gruppe des Wirkstoffes 1,5fachen Überschuß an dem O-Acylcarnitinylchlorid-Gemisch aus Stufe 2 versetzt. Nach 6stündigem Rühren des Reaktionsgemisches bei Raumtemperatur werden 20 ml wasserfreies Methanol zugesetzt, um überschüssiges Carnitinylchloridgemisch zu binden. Die Reaktionsmischung wird sodann weitere 5 Minuten gerührt. Anschließend werden die flüchtigen Bestandteile abdestilliert. Der erhaltene Rückstand kann ohne weitere Reinigung direkt in den erfindungsgemäßen Haarbehandlungsmitteln eingesetzt werden.

**O-Acetylcarnitinyl-cholesterin**

Die Charakterisierung erfolgte durch UV-Spektroskopie mit einem Spektrometer der Firma Perkin-Elmer, Typ Lambda 16 (siehe Abbildung 1).

UV-Spektrum: $\lambda$ = 202-205 nm; A = 1,27-1,28

Alle in der vorliegenden Anmeldung genannten Prozentangaben stellen, soweit nicht anders angegeben, Gewichtsprozente dar.

**Patentansprüche**

1. Wirkstoffderivate der Formel (I)

$$\begin{array}{l} \quad\;\; R^1 \\ \quad\;\; | \\ [R^2 - N^+ - Y - COOR]\ A^- \qquad (I), \\ \quad\;\; | \\ \quad\;\; R^3 \end{array}$$

mit $R^1$, $R^2$ und $R^3$ unabhängig voneinander gleich einem unsubstituierten oder substituierten Alkylrest, Y gleich einem substituierten oder unsubstituierten Alkylrest, R gleich einem Wirkstoffradikal, das vor der Esterbildung mindestens eine OH-Gruppe im Molekül aufweist und $A^-$ gleich einem Anion.

2. Wirkstoffderivat nach Anspruch 1, dadurch gekennzeichnet, daß $R^1$, $R^2$ und $R^3$ unabhängig voneinander einen $C_1$- bis $C_6$- Alkylrest darstellen.

3. Wirkstoffderivat nach Anspruch 1 oder 2, dadurch gekennzeichnet, daß $R^1$, $R^2$ und $R^3$ unabhängig voneinander einen Methyl-, Ethyl-, Propyl-, Isopropyl- oder Butylrest darstellen.

4. Wirkstoffderivat nach einem der Ansprüche 1 bis 3, dadurch gekennzeichnet, daß Y einen unsubstituierten $C_1$- bis $C_6$- Alkylrest oder einen mit einer oder mehreren Hydroxy-, Amino-, Acyl- oder quaternären Ammoniumgruppen substituierten $C_2$- bis $C_{10}$- Alkylrest darstellt.

5. Wirkstoffderivat nach einem der Ansprüche 1 bis 4, dadurch gekennzeichnet, daß $R^1$, $R^2$ und $R^3$ gleichzeitig einen Methylrest darstellen und Y gleich - $CH_2$-CH(OH)-$CH_2$- oder -$CH_2$-CH(OOC-$CH_3$)-$CH_2$- oder -$CH_2$- ist.

6. Wirkstoffderivat nach einem der Ansprüche 1 bis 5, dadurch gekennzeichnet, daß der Wirkstoff des Restes R ausgewählt ist aus mindestens eine OH-Gruppe enthaltenden Pflegestoffen, Vitaminen, Duftstoffen, Konservierungsstoffen, fungiziden oder bakteriziden Wirkstoffen, Lipiden oder Farbstoffen.

7. Verfahren zur Herstellung von Wirkstoffderivaten der Formel (I) bei dem die Verbindung der Formel (II)

$$\begin{array}{l} \quad\;\; R^1 \\ \quad\;\; | \\ [R^2 - N^+ - Y - COOH]\ A^- \qquad (II), \\ \quad\;\; | \\ \quad\;\; R^3 \end{array}$$

durch Überführung in das entsprechende Säurechlorid aktiviert und sodann mit einem Wirkstoff, welcher mindestens eine OH-Gruppe im Molekül aufweist, verestert wird.

8. Mittel zur Haarbehandlung, dadurch gekennzeichnet, daß es mindestens ein Wirkstoffderivat der Formel (I) gemäß einem der Ansprüche 1 bis 6 enthält.

9. Mittel nach Anspruch 8, dadurch gekennzeichnet, daß es einen pH-Wert von 2 bis 7 aufweist.

10. Mittel nach Anspruch 8 oder 9, dadurch gekennzeichnet, daß es in Form eines Zweikomponentenpräparates, bei dem das Wirkstoffderivat der Formel (I) getrennt von den übrigen Bestandteilen abgepackt wird, vorliegt.

11. Verfahren zum Behandeln von Haaren, dadurch gekennzeichnet, daß man eine für die Haarbehandlung ausreichende Menge des Haarbehandlungsmittels gemäß einem der Ansprüche 8 bis 10 auf das Haar aufträgt und nach einer Einwirkungszeit von 1 bis 60 Minuten bei 15 bis 50 Grad Celsius mit Wasser ausspült, gegebenenfalls mit einem Shampoo wäscht und trocknet.

12. Verfahren zum Behandeln von Haaren, dadurch gekennzeichnet, daß man eine ausreichende Menge des Haarbehandlungsmittels gemäß einem der Ansprüche 8 bis 10 auf das Haar aufträgt, das Haarbehandlungsmittel bei 15 bis 50 Grad Celsius 1 bis 60 Minuten einwirken läßt, sodann das Haar 1 bis 15 Minuten mit einer alkalischen Lösung (pH > 7) behandelt und anschließend mit Wasser ausspült, gegebenenfalls mit einem Shampoo wäscht und trocknet.

13. Verfahren nach Anspruch 12, dadurch gekennzeichnet, daß als alkalische Lösung eine 0,5- bis 10prozentige wäßrige Ammoniak-, Guanidin-, Ammoniumcarbonat-, Ammoniumhydrogencarbonat-, Alkalicarbonat- oder Alkalihydrogencarbonatlösung verwendet wird.

Abbildung 1

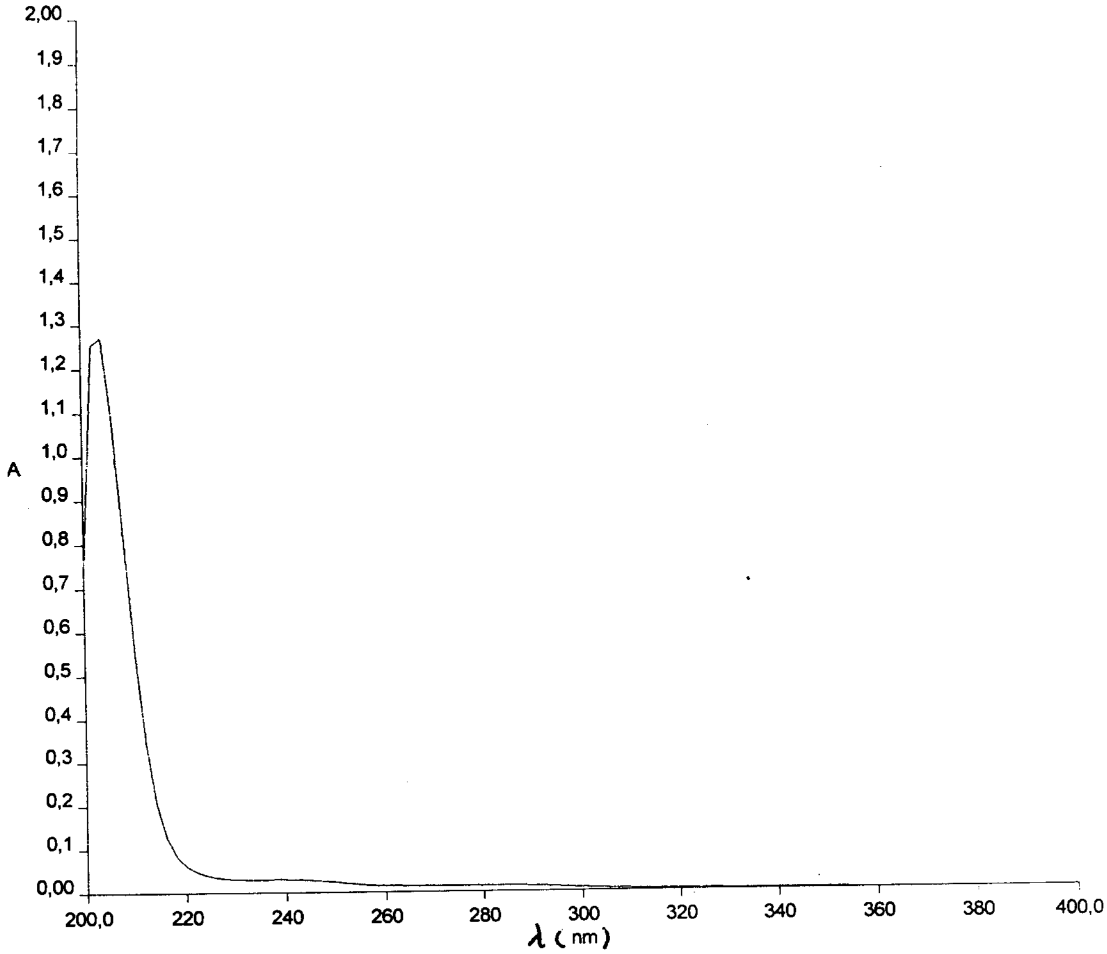


Beschreibung: Acetylcarnitincholesterin(20,8mg/100ml EtOH)

Registriergeschwindigkeit: 240.00 nm/min

Spaltbreite: 1,0000 nm

Spektrometer: LAMBDA 16 (Perkin Elmer)

Glättungsintervall: 4,00 nm

Datenintervall: 2,0000 nm